# EUROPEAN PATENT SPECIFICATION

(11) **EP 1 556 696 B1**
(45) Date of publication and mention of the grant of the patent: **23.07.2014**
(21) Application number: 03809772.1
(22) Date of filing: 29.10.2003
(51) Int. Cl.: G01N 33/548

(54) **SUGAR BINDING SURFACE**
ZUCKER BINDENDE OBERFLÄCHE
SURFACE DE LIAISON DE SUCRES

(30) Priority: 30.10.2002 GB 0225197
(43) Date of publication of application: 27.07.2005
(73) Proprietor: Corning Incorporated, Corning, New York 14831 (US)
(72) Inventor: Short, Robert, Sheffield S1 4DP (GB); Buttle, David, Sheffield S10 2RX (GB); Day, Anthony, MCR Immunochemistry Unit, Oxford OX1 3QU (GB)
(74) Representative: Charlton, Peter John
(86) International application number: PCT/GB2003/004653
(87) International publication number: WO 2004/040308

(56) References cited:
- EP-A- 0 124 200
- WO-A-01/31339
- WO-A-01/45862
- WO-A-90/00343
- WO-A-94/10938
- WO-A-03/030958
- US-A- 4 326 532
- CHU P K ET AL: "Plasma-surface modification of biomaterials" MATERIALS SCIENCE AND ENGINEERING R: REPORTS, ELSEVIER SEQUOIA S.A., LAUSANNE, CH, vol. 36, no. 5-6, 29 March 2002 (2002-03-29), pages 143-206, XP004343705 ISSN: 0927-796X

## Description

The invention relates to a method for the immobilisation of carbohydrates onto a surface, including substrates and products comprising said surfaces.

Carbohydrates are organic compounds derived from carbon, hydrogen, and oxygen, they are hydrates of carbon, having the general chemical formula Cₓ(H₂0)_{y}. The carbon atoms are normally in a linear chain and can be named by reference to the length of the these chains, for example, a carbohydrate with five carbon atoms is referred to as a pentose. Sugar, starch and cellulose are types of carbohydrate. Sugars are often referred to as simple carbohydrates and examples include glyceraldehyde (C₃H₆O₃), glucose (C₆H₁₂O₆) and sucrose (C₁₂H₂₂O₁₁).

Polymers of sugars are referred to as saccharides. Where three, four or many sugars are linked together they are referred to as tri-, tetra- or polysaccharides (glycans), where these are composed of the same sugars they are referred to as a homopolysaccharides. If they are of different sugars they are referred to as heteropolysaccharides.

The most abundant heteropolysaccharides in the body are the glycosaminoglycans (GAGs) also referred to as anionic mucopolysaccharides. These molecules are long unbranched polysaccharides containing a repeating disaccharide unit. The disaccharide units contain either of two modified sugars galactosamine (Gal) or glucosamine (Glc) and an uronic acid such as glucuronate or iduronate. GAGs are highly negatively charged molecules, with extended conformation that imparts high viscosity to a solution containing the GAGs. GAGs are located primarily on the surface of cells or in the extracellular matrix. The GAGs of physiological significance are hyaluronan, dermatan sulfate, chondroitin sulfate, heparin, heparan sulphate, and keratan sulphate.

Heparin, and the structurally related heparan sulfate, is a heterogeneous group of straight-chain glycosaminoglycans having anticoagulant properties. Although others may be present, the main sugars occurring in heparin are; (1) α-L-iduronic acid 2-sulfate, (2) 2-deoxy-2-sulfamino-α-D-glucose 6-sulfate, (3) β-D-glucuronic acid, (4) 2-acetamido-2-deoxy-α-D-glucose, and (5) α-L- iduronic acid. These sugars are present in decreasing amounts, usually in the order (2) > (1) > (4) > (3) > (5), and are joined by glycosidic linkages, forming polymers of varying sizes.

A number of methods for immobilising carbohydrates onto surfaces have been previously described.

US 6,180,769 discloses a method for linking negatively charged macrobiomolecules, such as glycosaminoglycans (GAGs) onto plastics. The method comprises contacting the macromolecules and the plastics with a non chaotropic solution containing a salt, preferably a salt belonging to the Hofmeister series of salts (e.g. NaCl, KCI, LiCl), in an amount of at least 20% of its saturation concentration.

A method of immobilising a complex of one or more labelled carbohydrates onto a solid surface such as glass is disclosed in US 5,641,390. In this method the labelled carbohydrates are derivatized in a substantially hydrophobic solvent system using conventional derivatisation reagents such as aminomethylfluorescein and 2-aminobenzoic acid (anthranilic acid). The labelled complex is then bound to the solid-phase and any contaminants, such as excess labelling reagent, removed by washing with a hydrophobic solvent such as butanol.

Both of these methods involve multiple chemical steps which can be both time-consuming and costly. Furthermore the use of harsh reagents, such as high salt concentrations, acids and solvents, have the potential to damage the structure of the bound carbohydrate and also have a number of associated safety issues. The number and types of carbohydrate that can be bound to a surface is limited due to need to tailor the reagents and conditions used to the type of complex to be formed.

A method of immobilising carbohydrates to a biosensor in order to generate a detectable signal via the specific binding of a protein, virus or cell is disclosed in US2001017270. The complete carbohydrate, or fragments thereof, referred to as oligosaccharides, can be modified at their reducing end with an O-, N-, C- or S-glycosidically bound aglycon, which can be an aliphatic or an aromatic compound, an amino-acid, peptide- or protein molecule or derivative thereof. Examples of aglycons include OEtSEtCONHNH₂, and -OetSPhMH₂. The binding of the aglycon to the surface of the biosensor can be effected directly, via proteins, such as bovine serum albumin, or via a chemical linkage which has been adsorbed or covalently bound to the surface. Such chemical structures include carboxyl-, sulfonate, cyanate, epoxy-, aldehyde groups or other groups suitable for chemical conjunction with for example an amine or thiol group in the aglycon. With the carbohydrate being modified with extra chemical groups in order to aid binding, one important consideration is that the carbohydrate is not bound in its native conformation and this may result in altered binding specificities and kinetics.

To overcome the problems associated with the current methods for the immobilisation of carbohydrates, several studies have investigated the immobilisation of carbohydrates using plasma polymerisation of compounds onto surfaces in order to provide modified surfaces which bind carbohydrate species. These studies have focussed on the immobilisation of carbohydrates to the surfaces of medical devices, or surgically-implantable materials, to reduce protein adsorption (fouling) to these surfaces.

Plasma polymerisation is a technique which allows an ultrathin (e.g. ca.200nm) cross linked polymeric film to be deposited on substrates of complex geometry and with controllable chemical functionality. As a consequence, the surface chemistry of materials can be modified, without affecting the bulk properties of the substrate so treated. Plasmas or ionised gases are commonly excited by means of an electric field. They are highly reactive chemical environments comprising ions, electrons, neutrals (radicals, metastables, ground and excited state species) and electromagnetic radiation. At reduced pressure, a regime may be achieved where the temperature of the electrons differs substantially from that of the ions and neutrals. Such plasmas are referred to as "cold" or "non-equilibrium" plasmas. In such an environment many volatile organic compounds (e.g. volatile alcohol containing compounds, volatile acid containing compounds, volatile amine containing compounds, or volatile hydrocarbons, neat or with other gases, e.g. Ar) have been shown to polymerise (H.K. Yasuda, Plasma Polymerisation, Academic Press, London 1985) coating both surfaces in contact with the plasma and those downstream of the discharge. The organic compound is often referred to as the "monomer". The deposit is often referred to as "plasma polymer". The advantages of such a mode of polymerisation potentially include: ultra-thin pin-hole free film deposition; plasma polymers can be deposited onto a wide range of substrates; the process is solvent free and the plasma polymer is free of contamination.

Under conditions of low power, plasma polymer films can be prepared which retain a substantial degree of the chemistry of the original monomer. For example, plasma polymerised films of acrylic acid contain the carboxyl group (O'Toole L., Beck A.J., Short R.D., Macromolecules, 1996, 29, 5172-5177). The low power regime may be achieved either by lowering the continuous wave power, or by pulsing the power on and off (Fraser S., Barton D., Bradley J.W., Short R.D., J. Phys. Chem. B., 2002, 22(106), 5596-5608).

Co-polymerisation of one or more compounds having functional groups with a hydrocarbon allows a degree of control over the surface functional group concentrations in the resultant plasma copolymer (PCP) (Beck A.J., Jones F.R., Short R.D., Polymer, 1996, 37(24), 5537-5539). Suitably, the monomers are ethlenically unsaturated. The functional group compound may be unsaturated carboxylic acid, alcohol or amine, for example, whilst the hydrocarbon is suitably an alkene. An example of plasma copolymerisation is the mixing of acrylic acid with octadiene in varying proportions of: 100 (aa) : 0 (oct.), 90: 10, 80: 20, and so forth until 0:100. Alternatively, the functionalised monomer may be allyl amine. By plasma polymerisation, it is also possible to deposit ethylene oxide-type molecules (eg. tetraethyleneglycol monoallyl ether) to form 'non-fouling' surfaces (Lopez G.P., Ratner B.D., Tidwell C.D., Haycox C.L., Rapoza R.J., Horbett T.A., J. Biomed. Mater. Res. 1992, 26, 425-439). Addition of a small amount of functionalised monomer (e.g. acrylic acid) introduces functional groups for subsequent chemistry/binding etc, in an essentially non-fouling surface. It is also possible to deposit perfluoro-compounds (i.e. perfluorohexane, hexafluoropropylene oxide) to form hydrophobic/superhydrophobic surfaces (Haque Y., Ratner B.D., J. Appl. Polym. Sci., 1986, 32, 4369-4381). This technique is advantageous because the surfaces have unique chemical and physical characteristics. Moreover, the surface wettability, adhesion and frictional wear characteristics of the substrate can be modified in a controllable and predictable manner.

Thin polymeric films can be obtained from the plasmas of volatile organic compounds (at reduced pressure of 1-10⁻³ mbar and ideally less than 100°C). In plasma polymer deposition, there is generally extensive fragmentation of the starting compound or ionised gas and a wide range of the resultant fragments or functional groups are undesirably incorporated into the deposit. By employing a low plasma input power (low plasma power/monomer flow rate ratio) it is possible to fabricate films with a high degree of functional group retention. Typically, using the composite ratio of W/FM, as described by Yasuda (Plasma Polymerisation, Academic Press, 1985) the power loading should be <10⁹ J/kg to achieve functional group retention in plasma polymers. (W = Power (J/min), F = Flow rate (mol/min), M = average molecular mass (kg/mol). More typically, this ratio will be ca. 1 x 10⁷ J/kg, or even less, for high levels of functional group retention. However, other relatively low ratios may be used and are known to those skilled in the art. Alternatively, plasma polymer deposits may be formed by pulsing the plasmas or ionised gases. Plasmas are formed either from single monomer species or in combination with other organic molecules

Although several studies have investigated the immobilisation of carbohydrates to plasma polymerisation treated surfaces, they have failed to demonstrate the immobilisation of the carbohydrates in their native form. In WO94/10938 (Case Western Reserve University), WO90/00343 (Cardiopulmonics Inc.) and WO01/45862 (Innerdyne Inc.), carbohydrate immobilisation is based on the use of a chemical adaptor, linker or spacer to couple the carbohydrate to the plasma polymerisation treated surface. As such, the carbohydrate is not bound to the surface in its native form but rather is chemically modified prior to binding possibly altering the structure or binding specificity or functionality of the carbohydrate molecule.

EP1048304 (Novartis) describes the covalent immobilisation of polysaccharides coatings to a plasma polymer surface of n-heptyl amine. This document addresses the provision of coatings that have a reduced rate of fouling by proteins (for biomedical devices, particularly contact lenses) and thus reduce rates of adverse biomedical consequences. Thus the covalent binding of polysaccharides to a surface appears to be essential for long-term patency in biomedical devices. The covalent binding of the polysaccharide involves first the covalent binding of a difunctional link molecule to the plasma polymer surface and then the covalent binding of the "free" unbound end of this link molecule to the polysaccharide. The polysaccharide is not bound at a single point to the surface, but at a number of points, and cannot therefore adopt a confirmation approaching its native form (e. g. in solution). In this way, characteristics of the carbohydrate, such as its binding functionality/activity, are likely to be affected.

WO94/10938 provides a method for surface modifying a substrate to provide the substrate with anticoagulant activity and resistance to the deposition of plasma proteins; and the resulting substrate.

We herein describe a method which overcomes the problems associated with the current methods for the immobilisation of carbohydrates.

In a first aspect, the invention provides a method to immobilise at least one type of carbohydrate molecule comprising the steps of:
i) providing a polymerizable allylamine monomer source;
ii) creating a plasma of said polymerizable allylamine monomer;
iii) coating a surface with said plasma to provide a plasma polymer coated surface; and
iv) contacting said polymer coated surface with a carbohydrate solution comprising at least one type of biologically active carbohydrate molecule wherein said carbohydrate molecule is a glycosaminoglycan selected from the group consisting of hyaluronan, dermatan sulphate, chondroitin sulphate, heparin sulphate or keratan sulphate.

A method to immobilise at least one type of carbohydrate molecule comprising contacting a surface with a plasma of at least one monomer to provide a plasma polymer coated surface and contacting said polymer surface with a carbohydrate molecule wherein the carbohydrate molecule is in its native form is described.

The "native form" is intended to include carbohydrates which are not physically or chemically modified, or carbohydrates modified to the extent that the functionality, for example substrate or binding specificity or biological activity, is unaffected. The skilled person will appreciate that minor modifications to the carbohydrates are possible without altering the physical characteristics of the molecule. However, it is preferred that the carbohydrate molecule is bound to the plasma polymer surface without any modification to its native form.

It is preferred that in its native form, the carbohydrate can passively adsorb to the plasma polymer treated surface.

Preferably, the carbohydrates are passively adsorbed to the plasma polymer surface.

Passive adsorption is intended to include, for example, non-covalent, electrostatic, hydrophilic or hydrophobic interactions between the carbohydrate and the plasma polymer surface.

A method to immobilise a carbohydrate molecule is described comprising the steps of :
i) providing a monomer source;
ii) creating a plasma of said monomer;
iii) coating a surface with said plasma to provide a plasma polymer coated surface; and
iv) contacting said polymer coated surface with at least one type of carbohydrate molecule, whereby said carbohydrate molecule is passively adsorbed to the polymer coated surface.

In the method of the invention the carbohydrate is provided as a solution comprising at least one carbohydrate molecule.

In a preferred method, the carbohydrate is provided in a solution of physiological pH, for example, pH 6 to 8. More preferably, the pH is between 6.8 and 7.4.

In an alternative preferred method, the carbohydrate molecule is negatively charged. The carbohydrate may be negatively charged under acid conditions, for example at pH 2.0, 3.0, 4.0, 5.0 or 6.0.

In a preferred method said surface comprises a polymer comprising a nitrogen content of at least 2%. Preferably said nitrogen content is 2-20%. Alternatively said nitrogen content is greater than 20%. The percentages refer to the percent of nitrogen atoms in the surface. For example 20% nitrogen means that 20 of every one hundred atoms in the plasma polymer is nitrogen.

The nitrogen content of a surface is determined by methods herein disclosed and are known in the art. For example, percent nitrogen maybe measured by x-ray photoelectron spectroscopy (XPS).

Polymerizable monomers that may be used in the described methods preferably comprise unsaturated organic compounds such as olefinic amines, halogenated olefins, olefinic carboxylic acids and carboxylates, olefinic nitrile compounds, oxygenated olefins and olefinic hydrocarbons. Such olefins include vinylic and allylic forms. The monomer need not be olefinic, however, to be polymerizable. Cyclic compounds such as cyclohexane, cyclopentane and cyclopropane are commonly polymerizable in gas plasmas by glow discharge methods. Derivatives of these cyclic compounds, such as 1, 2- diaminocyclohexane for instance, are also commonly polymerizable in gas plasmas.

Particularly preferred are polymerizable monomers containing hydroxyl, amino or carboxylic acid groups. Of these, particularly advantageous results have been obtained through use of allylamine. Mixtures of polymerisable monomers may be used. Additionally, polymerisable monomers may be blended with other gases not generally considered as polymerisable in themselves, examples being argon, nitrogen and hydrogen. The polymerisable monomers are preferably introduced into the vacuum chamber in the form of a vapour. Polymerisable monomers having vapour pressures less than 5 x 10⁻³mbar are not generally suitable for use in the practice of the described methods. The vapour pressure of monomers may be elevated by heating of the monomer.

Polymerisable monomers having vapour pressures of at least 6.6 x 10⁻²mbar at ambient room temperature are preferred. Where monomer grafting to plasma polymerisate deposits is employed, polymerisable monomers having vapour pressures of at least 5x10⁻³ mbar at ambient conditions are particularly preferred.

To maintain desired pressure levels, especially since monomer is being consumed in the plasma polymerisation operation, continuous inflow of monomer vapour to the plasma zone is normally practised. Continuous removal of excess gases is accomplished by simultaneously pumping through the vacuum port to a vacuum source. Since some non-polymerisable gases are often evolved from glow discharge gas plasmas, it is advantageous to control gas plasma pressure at least in part through simultaneous vacuum pumping during plasma polymerisate deposition on a substrate in the described methods.

Examples of typical monomers include, fully saturated and unsaturated amine compounds up to 20 carbon atoms. More typically 2-8 carbons. Ethylenically unsaturated compounds (especially primary, secondary or tertiary amines) including allylamine. Saturated monomers include methylamine, propylamine, heptylamine and diaminopropane.

In a further preferred method said polymer comprises an amine copolymer. The co-polymer is prepared by the plasma polymerisation of an organic amine with a saturated (alkane) or unsaturated (alkene, diene or alkyne) hydrocarbon. The hydrocarbon would be of up to 20 carbons (but more usually of 4- 8). Examples of alkanes are butane, pentane and hexane. Examples of alkenes are butene and pentene. An example of a diene is 1-7 octadiene. The co-monomer may also be aromatic-containing e.g. styrene.

Co-plasma polymerisation may be carried out using any ratio of amine : hydrocarbon, but will be typically using an amine : hydrocarbon ratio between the limits of 100 (amine):0(hydrocarbon) to 20 (amine):80 (hydrocarbon) and any ratio between these limits.

The glow discharge through the gas or blend of gases in the vacuum chamber may be initiated by means of an audiofrequency, a microwave frequency or a radiofrequency field transmitted to or through a zone in the vacuum chamber. Particularly preferred is the use of a radiofrequency (RF) discharge, transmitted through a spatial zone in the vacuum chamber by an electrode connected to an RF signal generator. A rather broad range of RF signal frequencies starting as low as 50 kHz may be used in causing and maintaining a glow discharge through the monomer vapour. In commercial scale usage of RF plasma polymerisation, an assigned radiofrequency of 13.56 MHz may be more preferable to use to avoid potential radio interference problems as with examples given later.

The glow discharge need not be continuous, but may be intermittent in nature during plasma polymerisate deposition. Or, a continuous glow discharge may be employed, but exposure of a substrate surface to the gas plasma may be intermittent during the overall polymerisate deposition process. Or, both a continuous glow discharge and a continuous exposure of a substrate surface to the resulting gas plasma for a desired overall deposition time may be employed. The plasma polymerisate that deposits onto the substrate generally will not have the same elemental composition as the incoming polymerisable monomer (or monomers). During the plasma polymerisation, some fragmentation and loss of specific elements or elemental groups naturally occurs. Thus, in the plasma polymerisation of allylamine, nitrogen content of the plasma polymerisate is typically lower than would correspond to pure polyallylamine. Similarly, in the plasma polymerisation of acrylic acid, carboxyl content of the plasma polymerisate is typically lower than would correspond to pure polyacrylic acid. Exposure time to either of these unreacted monomers in the absence of a gas plasma, as through intermittent exposure to a glow discharge, allows for grafting of the monomer to the plasma polymerisate, thereby increasing somewhat the level of the functional group (amine or carboxylic acid) in the final deposit. Time intervals between plasma exposure and grafting exposure can be varied from a fraction of a second to several minutes.

In a preferred method of the invention the plasma polymer is deposited from a plasma of W/FM of <10⁹ J/kg and ideally < 10⁸ J/Kg and more ideally < 10⁷ J/Kg, where W = power(J/min), F = flow rate (mol/min) and M = average molecular mass (kg/mol).

In the method of the invention the carbohydrate molecule comprises a glycosaminoglycan.

In a preferred method said carbohydrate is a sulphated biomolecule, preferably highly sulphated.

In the method of the invention said glycosaminoglycan is selected from the group consisting of: hyaluronan; dermatan sulfate; chondroitin sulphate; heparin; heparan sulphate; or keratan sulphate.

Passive adsorption involves the incubation of a surface with the carbohydrate in solution, such that the carbohydrate binds with the surface. The binding should be sufficiently strong that the polysaccharide is immobilised to the surface to the extent that it cannot be desorbed by washing, or by the typical processes carried out in biochemical or chemical assays. The immobilised polysaccharide should be bound in such a manner that it retains its (native) biological activity, as demonstrated by binding with target molecules that it would normally bind with it in solution.

Passive adsorption to plasma polymer surfaces may be carried out from a solution containing polysaccharide, over a range of pH. Preferably the pH is from 3 to 11, for example pH 4 to 10, 5 to 9, 6 to 8 or 7.

Passive adsorption to plasma polymer surfaces may be carried out from a solution containing polysaccharide concentrations (1ng/ml - 10 ng/ml 10-100ng/ml, 100 - 1000 ng/ml, or even microgram quantities per ml [1 - 10 µg/ml]). Adsorption is most likely (but not exclusively) to be carried out in the temperature range of 20-37.5°C.

Adsorption may be carried out from phosphate buffered saline or a solution of physiological ionic strength.

It is well known to those skilled in the art that the adsorption of specific polysaccharides, for example polysaccharides carrying a high net negative charge (e.g. sulphated GAGs e.g. heparin) to plastic surfaces is difficult to achieve. Plasticware which is available for biochemical and chemical assays (e.g. culture dishes, 96 well microtitre plates etc.) is typically manufactured from polystyrene (although it may be surface treated to improve binding properties). Surface treatments may include corona, plasma, acid or alkaline rinses, and flame. These treatments introduce a range of new surface functionalities into the plastic, mainly oxygen (alcohols, ethers, carbonyls and carboxyls, as well as peroxides). But, alone, these functionalities do not promote the passive adsorption of negatively charged molecules.

In assays, it is preferred that the polysaccharide is adsorbed pure. Moreover it is preferred that the polysaccharide is not contaminated (e.g. with albumin or salts), or that the immobilisation surface is modified (for example by the binding of a first biomolecule (for example, albumin) that will in turn bind the polysaccharide.

In a further preferred method of the invention said surface is part of a biosensor.

It will be apparent that biosensors maybe fabricated by the provision of a carbohydrate coated surface to allow the detection of biomolecules in a sample which bind, either directly or indirectly, carbohydrate molecules presented at the surface of the biosensor. The plasma polymerisation method allows the formation of a homogeneous surface which presents the immobilised carbohydrate in its native form thereby facilitating sensitive detection of a molecule present in a sample.

In a further preferred method of the invention said surface is part of a therapeutic vehicle.

Therapeutic vehicle includes means to deliver cells to a wound and includes, by example: valves (e.g. heart valves); prosthesis; implant; matrix; stent; biodegradable matrix; polymeric film; wound dressings e.g. bandages; gauze; tape; or plaster casts. Implantable devices show increased integrity and stability when associated with glycosaminoglycans, see WO00/64371. The present invention describes a vehicle comprising a surface with a plasma polymer coating of glucosaminoglycan which has improved properties when compared to prior art vehicles. Moreover, wound dressings coated with glucosaminoglycans show chemotactic properties, see US4837024, which attract cells involved in tissue repair (e.g. fibroblasts, endothelial cells) which enhance healing. The coating of dressings with glucosaminoglycans, in particular, heparin, heparan sulphate or alginate.

A further preferred method wherein said surface provides a cell culture surface is also described.

In a yet further preferred method of the invention said surface is part of a device wherein said device is used in the collection of biological samples from an animal, preferably a human.

Devices used in the collection of, for example blood or serum samples, include syringes, blood collection bags, plastic bottles and the like, which are coated with heparin to prevent blood contained therein from clotting. Also included are devices used in kidney dialysis, for example dialysis tubing.

In a yet still further method of the invention said surface is part of an affinity purification matrix.

Affinity purification is a well known method to isolate biological molecules which bind a molecule which is immobilised on an inert matrix. The immobilised molecule is a protein (e.g. a ligand, receptor, antibody) which has affinity for a target molecule in a complex, often unfractionated sample. The surfaces obtainable by the method according to the invention would have particularly useful properties in this respect because the immobilised carbohydrates would have a high probability of retaining their native structure thereby facilitating the binding of proteins which have specificity for a particular glucosaminoglycan.

In a further preferred method of the invention said surface is part of a microarray.

Genomics analysis involves the analysis of sequence information (DNA, RNA or protein) typically generated from genome sequencing projects. Typically biomolecules immobilised for this purpose are referred to as arrays or microarrays. An array is a two-dimensional sheet to which is applied different biomolecules at different sites on the sheet. This facilitates the screening of the biomolecules in parallel and on a much smaller scale than conventional solid phase assays.

Typically biomolecules are immobilised by chemical coupling or adsorption. Currently arrays of biomolecules are made by depositing aliquots of sample under conditions which allow the molecules to bind or be bound to the array surface. Alternatively, or in addition, biomolecules may be synthesised at the array surface and directly or indirectly immobilised. The number of different samples that are applied to a single array can reach thousands.

The application of samples to form an array can be facilitated by the use of "array printers", (for example see Gene Expression Micro-Arrays, A New Tool for Genomics, Shalon, D, in Functional Genomics, IBC library series; Southern EM, DNA Chips: Analysing Sequence by Hybridisation to Oligonucleotides on a Large Scale, Trends in Genetics, 12: 110-5, 1996). The analysis of micro-arrays is undertaken by commercially available "array readers" which are used to interpolate the data generated from the array, for example as disclosed in US5, 545, 531. Arrays are typically made individually and used only once before being disposed of. Therefore, it is highly desirable to produce arrays which are manufactured to a high degree of reproducibility and with minimum error.

An array comprising a surface obtained by the method of the invention would allow the binding of proteins which bind, for example glucosaminoglycans. An array maybe fabricated to contain different types of glucosaminoglycans to facilitate the identification, from complex mixtures, of proteins with particular specificities and/or affinities for a particular glucosaminoglycan or combination of glucosaminoglycan.

According to an aspect of the invention there is provided a biosensor comprising a surface obtainable by the method according to the invention.

According to a further aspect of the invention there is provided a therapeutic vehicle comprising a surface obtainable by the method according to the invention.

According to a further aspect of the invention there is provided a sample collection device comprising a surface obtainable by the method according to the invention.

According to a yet further aspect of the invention there is provided an affinity purification matrix comprising a surface obtainable by the method according to the invention.

According to a further aspect of the invention there is provided a microarray comprising a surface obtainable by the method according to the invention.

A cell culture system comprising a surface obtainable by the method according to the invention is also described.

An embodiment of the invention will now be described by example only and with reference to the following materials, methods and figures:
Figure 1 is a diagrammatic illustration of a plasma apparatus; and
Figure 2: Binding of Heparin to allylamine plasma polymer coated plate.
Figure 3: Binding of different heparin preparations to allylamine plasma polymer coated plate.
Figure 4: Binding of KC to heparin on allylamine plasma polymer coated plate.

### Materials and Methods

### Plasma Polymerisation

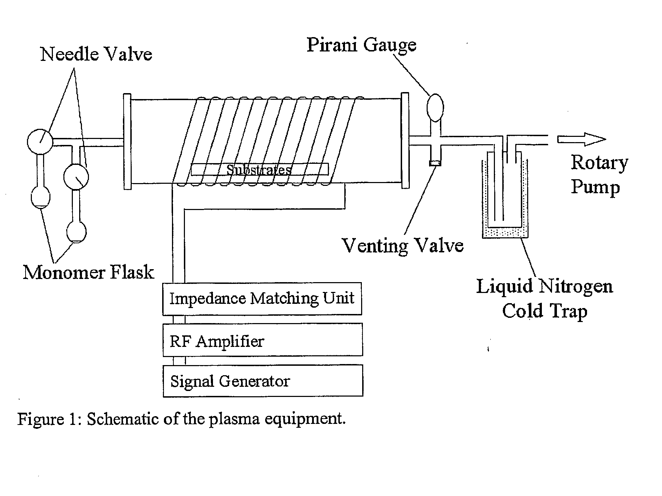

Plasma polymerisation was carried out onto 96-well microtiter plates using allylamine as a monomer. An RF (13.56MHz) power of less than 10W was used with a flow rate between 1-5 cm³ₛₜₚmin⁻¹ and a reactor pressure of around 2x10⁻²mbar. The chemical nature of the deposited film was analysed by X-Ray Photoelectron Spectroscopy (XPS). A schematic diagram of the plasma apparatus is shown in Figure 1.

### Adsorption of Heparin

Heparin was adsorbed onto both allylamine coated and uncoated (Manufacturers proprietary treatment) overnight from PBS at room temperature. Following standard ELISA methods, the unbound heparin was washed from the surfaces, and the remaining bound molecules were detected using a biotinylated detector molecule (i.e., the Link module from human TSG-6) (Mahoney et al., J. Biol. Chem. 276, 22764-22771 (2001); Parkar & Day FEBS Lett. 410, 413-417 (1997).

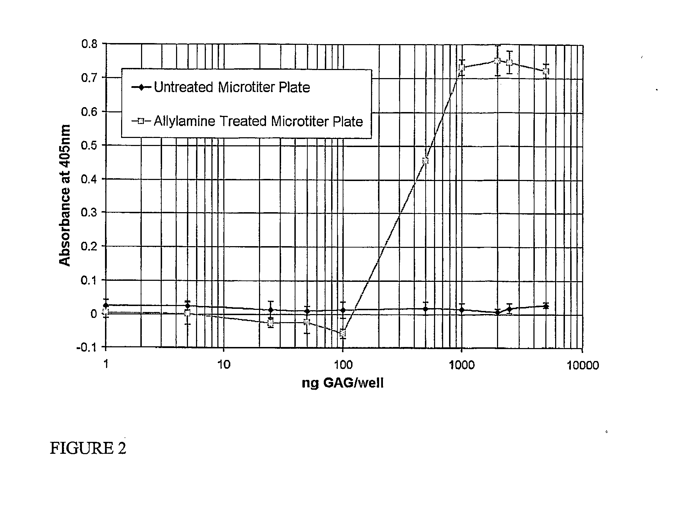

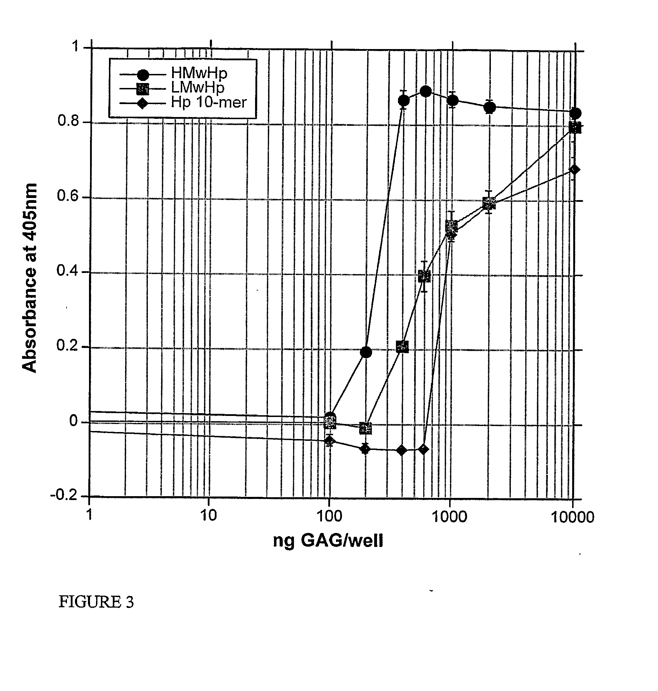

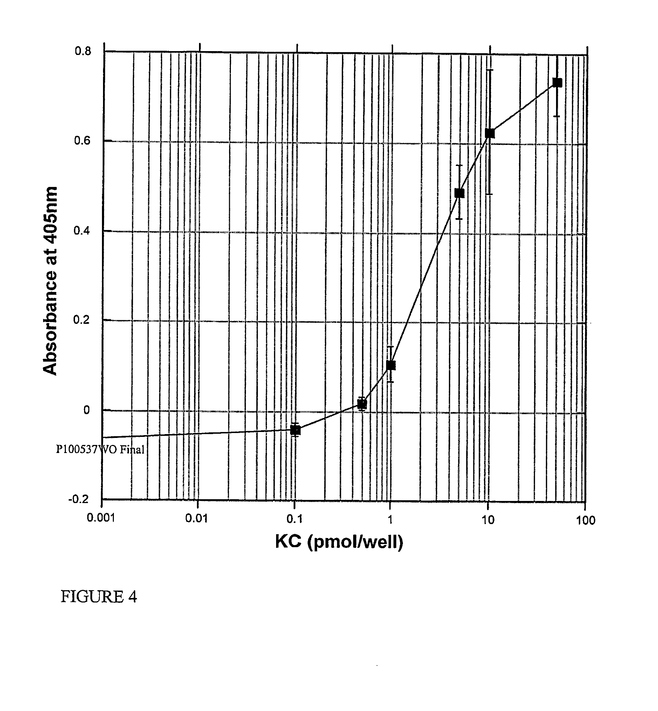

Colour was developed and measured using a plate reader in the usual manner. The results of averaging four separate measurements of adsorption onto untreated and allylamine treated plates, over the range of concentration is shown in figure 2. Figure 3 compares three different preparations of heparin (i.e., high Mr (HMw Hp; also used in the previous assay), low Mr (LMw Hp) and a defined decasaccharide (Hp 10-mer)) using the same detection system. Figure 4 shows an assay with a different detector protein (the mouse chemokine KC; where binding was determined using a biotinylated antibody), at a range of concentrations, with HMw Hp coated at 500 ng/well. The binding assays in figures 2 and 3 were conducted at pH 6.0, whereas, the experiment in figure 4 was performed at pH 7.2.

## Claims

1. A method to immobilise at least one type of carbohydrate molecule comprising the steps of :
i) providing a polymerizable allylamine monomer source;
ii) creating a plasma of said polymerizable allylamine monomer;
iii) coating a surface with said plasma to provide a plasma polymer coated surface; and
iv) contacting said polymer coated surface with a carbohydrate solution comprising at least one type of biologically active carbohydrate molecule wherein said carbohydrate molecule is a glycosaminoglycan selected from the group consisting of hyaluronan, dermatan sulphate, chondroitin sulphate, heparin, heparan sulphate or keratan sulphate.

2. A method according to claim 1 wherein the plasma polymer is deposited from a plasma of W/FM of < 10⁹ J/kg and ideally < 10⁸ J/kg and more ideally < 10⁷ J/kg, where W = power (J/min), F = flow rate (mol/min) and M = average molecular mass (kg/mol).

3. A method as claimed in claim 1 or claim 2 wherein the surface is part of a biosensor.

4. A method as claimed in claim 1 or claim 2 wherein the surface is part of a therapeutic vehicle.

5. A method as claimed in claim 1 or claim 2 wherein the surface is part of a device wherein said device is for use in the collection of biological samples from an animal, preferably a human.

6. A method as claimed in claim 1 or claim 2 wherein the surface is part of an affinity purification matrix.

7. A method as claimed in claim 1 or claim 2 wherein the surface is part of a microarray.

8. A biosensor comprising a surface obtained by a method as claimed in claim 1, 2 or 3.

9. A therapeutic vehicle comprising a surface obtained by a method as claimed in claim 1,2 or 4.

10. A sample collection device comprising a surface obtained by a method as claimed in claim 1, 2 or 5.

11. An affinity purification matrix comprising a surface obtained by a method as claim in claim 1, 2, or 6.

12. A microarray comprising a surface obtained by a method as claimed in claim 1, 2 or 7.

## Patentansprüche

1. Verfahren zum Immobilisieren mindestens eines Typs eines Kohlenhydratmoleküls, umfassend die folgenden Schritte:
i) Bereitstellen einer polymerisierbaren Allylaminmonomer-Quelle;
ii) Erzeugen eines Plasmas des polymerisierbaren Allylaminmonomers;
iii) Überziehen einer Oberfläche mit dem Plasma, um eine mit Plasmapolymer überzogene Oberfläche bereitzustellen; und
iv) Kontaktherstellen der mit Polymer überzogenen Oberfläche mit einer Kohlenhydratlösung, die mindestens einen Typ eines biologisch aktiven Kohlenhydratmoleküls umfasst, wobei das Kohlenhydratmolekül ein Glykosaminoglykan ist, das aus der Gruppe ausgewählt ist bestehend aus Hyaluronan, Dermatansulfat, Chondroitinsulfat, Heparin, Heparansulfat und Keratansulfat.

2. Verfahren nach Anspruch 1, wobei das Plasmapolymer abgeschieden wird von einem Plasma mit W/FM < 10⁹ J/kg und idealerweise < 10⁸ J/kg und noch besser < 10⁷ J/kg, mit W = Leistung (J/Min), F = Strömungsrate (Mol/Min) und M = mittlere Molekülmasse (kg/Mol).

3. Verfahren nach Anspruch 1 oder 2, wobei die Oberfläche Teil eines Biosensors ist.

4. Verfahren nach Anspruch 1 oder 2, wobei die Oberfläche Teil eines therapeutischen Vehikels ist.

5. Verfahren nach Anspruch 1 oder 2, wobei die Oberfläche Teil einer Vorrichtung ist, wobei die Vorrichtung zum Einsatz in der Entnahme biologischer Proben von einem Tier, vorzugsweise einem Menschen ist.

6. Verfahren nach Anspruch 1 oder 2, wobei die Oberfläche Teil einer Affinitätsreinigungsmatrix ist.

7. Verfahren nach Anspruch 1 oder 2, wobei die Oberfläche Teil eines Microarray ist.

8. Biosensor, der eine durch ein Verfahren nach einem der Ansprüche 1, 2 oder 3 erhaltene Oberfläche umfasst.

9. Therapeutisches Vehikel, das eine durch ein Verfahren nach einem der Ansprüche 1, 2 oder 4 erhaltene Oberfläche umfasst.

10. Probenentnahmevorrichtung, die eine durch ein Verfahren nach einem der Ansprüche 1, 2 oder 5 erhaltene Oberfläche umfasst.

11. Affinitätsreinigungsmatrix, die eine durch ein Verfahren nach einem der Ansprüche 1, 2 oder 6 erhaltene Oberfläche umfasst.

12. Microarray, das eine durch ein Verfahren nach einem der Ansprüche 1, 2 oder 7 erhaltene Oberfläche umfasst.

## Revendications

1. Procédé pour immobiliser au moins un type de molécule d'hydrate de carbone comprenant les étapes consistant à :
i) fournir une source de monomère allylamine polymérisable ;
ii) créer un plasma dudit monomère allylamine polymérisable ;
iii) recouvrir une surface avec ledit plasma pour former une surface recouverte de polymère plasma ; et
iv) mettre en contact ladite surface recouverte de polymère avec une solution d'hydrate de carbone comprenant au moins un type de molécule d'hydrate de carbone biologiquement active, dans lequel ladite molécule d'hydrate de carbone est un glycosaminoglycane choisi dans le groupe constitué de hyaluronane, sulfate de dermatane, sulfate de chondroïtine, héparine, sulfate d'héparane ou sulfate de kératane.

2. Procédé selon la revendication 1, dans lequel le polymère plasma est déposé d'un plasma de W/FM de < 10⁹ J/kg et idéalement < 10⁸ J/kg et plus idéalement < 10⁷ J/kg, où W = puissance (J/min), F = débit (mol/min) et M = masse moléculaire moyenne (kg/mol).

3. Procédé selon la revendication 1 ou 2, dans lequel la surface fait partie d'un biocapteur.

4. Procédé selon la revendication 1 ou 2, dans lequel la surface fait partie d'un véhicule thérapeutique.

5. Procédé selon la revendication 1 ou 2, dans lequel la surface fait partie d'un dispositif dans lequel ledit dispositif est destiné à être utilisé dans le prélèvement d'échantillons biologiques d'un animal, de préférence d'un humain.

6. Procédé selon la revendication 1 ou 2, dans lequel la surface fait partie d'une matrice de purification par affinité.

7. Procédé selon la revendication 1 ou 2, dans lequel la surface fait partie d'un microréseau.

8. Biocapteur comprenant une surface obtenue par un procédé selon la revendication 1, 2 ou 3.

9. Véhicule thérapeutique comprenant une surface obtenue par un procédé selon la revendication 1, 2 ou 4.

10. Dispositif de collecte d'échantillon comprenant une surface obtenue par un procédé selon la revendication 1, 2 ou 5.

11. Matrice de purification par affinité comprenant une surface obtenue par un procédé selon la revendication 1, 2 ou 6.

12. Microréseau comprenant une surface obtenue par un procédé selon la revendication 1, 2 ou 7.
